# EUROPEAN PATENT SPECIFICATION

(11) **EP 2 692 283 B1**
(45) Date of publication and mention of the grant of the patent: **12.07.2017**
(21) Application number: 13178147.8
(22) Date of filing: 26.07.2013
(51) Int. Cl.: A61B 3/10

(54) **Ophthalmologic apparatus and ophthalmologic method**
Ophthalmologische Vorrichtung und ophthalmologisches Verfahren
Appareil ophtalmologique et procédé ophtalmologique

(30) Priority: 30.07.2012 JP 2012167921; 30.07.2012 JP 2012167920; 30.07.2012 JP 2012167919
(43) Date of publication of application: 05.02.2014
(73) Proprietor: Canon Kabushiki Kaisha, Tokyo, Tokyo 146-8501 (JP)
(72) Inventor: Sakagawa, Wataru, Tokyo, Tokyo 146-8501 (JP); Umekawa, Kazuaki, Tokyo, Tokyo 146-8501 (JP); Itoh, Hiroshi, Tokyo, Tokyo 146-8501 (JP)
(74) Representative: TBK

(56) References cited:
- JP-A- 2003 290 146
- US-A1- 2011 157 554

## Description

### BACKGROUND OF THE INVENTION

### Field of the Invention

The present invention relates to an ophthalmologic apparatus for measuring ocular characteristics of an eye to be inspected or acquiring an image of the eye to be inspected, and to an ophthalmologic method for obtaining the ocular characteristics of the eye to be inspected.

### Description of the Related Art

In recent years, with the popularization of intraocular lenses (IOL) used for cataract surgeries, there are an increased number of an eye to be inspected having an intraocular lens implanted therein (IOL eye). An IOL has different characteristics than a crystalline lens in terms of its shape and material, the presence/absence of refractive power adjustability, and the like. Therefore, in order to inspect the IOL eye with high accuracy, an apparatus needs to acquire information on whether the eye to be inspected is the IOL eye.

There has been known a technology in which, in an ophthalmologic reflectometer, an inspector provides an input on whether the eye to be inspected is the IOL eye to the reflectometer and the reflectometer switches the function of a jog dial depending on the input (Japanese Patent No. 3244873). Therefore, for the IOL eye that generally tends to undergo miosis, the inspector may adjust the light amount of a fixation target with the jog dial.

There has been known a technology in which, in an ophthalmologic image acquiring apparatus, the apparatus determines, based on the color of the flare, whether the eye to be inspected is the IOL eye to switch the focusing method (Japanese Patent Application Laid-Open No. 2003-290146). Therefore, precise focusing can be performed for the IOL eye that generally tends to generate the flare.

There has also been known a technology in which, in an apparatus for measuring eye axial length, the apparatus determines, based on reflection signals from an anterior ocular segment of the eye to be inspected, whether the eye to be inspected is the IOL eye to use a more appropriate method for calculating the eye axial length (Japanese Patent Application Laid-Open No. 2011-136109).

Meanwhile, there has been known a technology in which, irrespective of whether the eye to be inspected is the IOL eye, for the purpose of preventing malfunction of an alignment operation, images in an on state and an off state of a light source are compared (Japanese Patent Application Laid-Open No. 2009-172155).

However, it has not been possible for the apparatus to use a bright spot image on a cornea to automatically determine whether the eye to be inspected is the IOL eye. Therefore, even with an apparatus that can obtain the bright spot image on the cornea, it has been necessary for the inspector to provide the input on whether the eye to be inspected is the IOL eye as in the configuration disclosed in Japanese Patent No. 3244873, and there has been a risk that the inspection fails due to a mistake or an input error of the inspector. There have also been problems in that such input operation places a burden on the inspector and in that the measurement time increases.

### SUMMARY OF THE INVENTION

The present invention has been made in view of the above-mentioned circumstances, and therefore is to provide an ophthalmologic apparatus or an ophthalmologic method that enables automatic determination of whether an eye to be inspected is an IOL eye by utilizing bright spot images on a cornea.

Note that, without limiting to the above-mentioned apparatus and method, providing actions and effects that are obtained by configurations described below in the "Description of the Embodiments" section and that cannot be obtained by the conventional technologies can also be regarded as another aspect of the present invention.

In order to solve the above-mentioned problems, an ophthalmologic apparatus according to one embodiment of the present invention includes: a projecting unit for projecting a light beam to an eye to be inspected; and a determining unit for determining whether or not a bright spot image based on a reflection light beam obtained by reflection of the light beam on the eye to be inspected is a bright spot image generated by an intraocular lens.

According to the present invention, the automatic determination on whether the eye to be inspected is the IOL eye is enabled by utilizing the bright spot images on the cornea. Therefore, the risk that the inspection fails due to the mistake or the input error of the inspector is reduced, and because the input operation is unnecessary, effects that the burden on the inspector is reduced and that the inspection time may be reduced may be obtained.

Further features of the present invention will become apparent from the following description of exemplary embodiments with reference to the attached drawings.

### BRIEF DESCRIPTION OF THE DRAWINGS

FIG. 1 is a diagram illustrating an example of an appearance of an ophthalmologic reflectometer according to a first embodiment of the present invention.
FIG. 2 is a diagram illustrating an example of an optical system arrangement of the first embodiment illustrated in FIG. 1.
FIG. 3 is a perspective view illustrating an example of an alignment prism stop of the first embodiment illustrated in FIG. 1.
FIG. 4 is a diagram illustrating an example of system blocks of the ophthalmologic reflectometer according to the first embodiment of the present invention.
FIG. 5 is a diagram illustrating an example of image forming positions of bright spot images obtained by the ophthalmologic reflectometer exemplified in the first embodiment.
FIGS. 6A and 6B are diagrams illustrating examples of anterior ocular segment images obtained by the ophthalmologic reflectometer exemplified in the first embodiment, of which FIG. 6A illustrates an anterior ocular segment image of a non-IOL eye, and FIG. 6B illustrates an anterior ocular segment image of an IOL eye.
FIG. 7 is a flow chart illustrating an example of IOL eye determination in ophthalmologic image acquisition according to the first embodiment of the present invention.
FIGS. 8A and 8B are diagrams illustrating examples of anterior ocular segment images obtained by an apparatus according to a second embodiment of the present invention, of which FIG. 8A illustrates an anterior ocular segment image of a non-IOL eye, and FIG. 8B illustrates an anterior ocular segment image of an IOL eye.
FIG. 9 is a flow chart illustrating an example of IOL eye determination in ophthalmologic image acquisition according to the second embodiment of the present invention.

### DESCRIPTION OF THE EMBODIMENTS

### [First embodiment]

The present invention is described in detail based on illustrated embodiments.

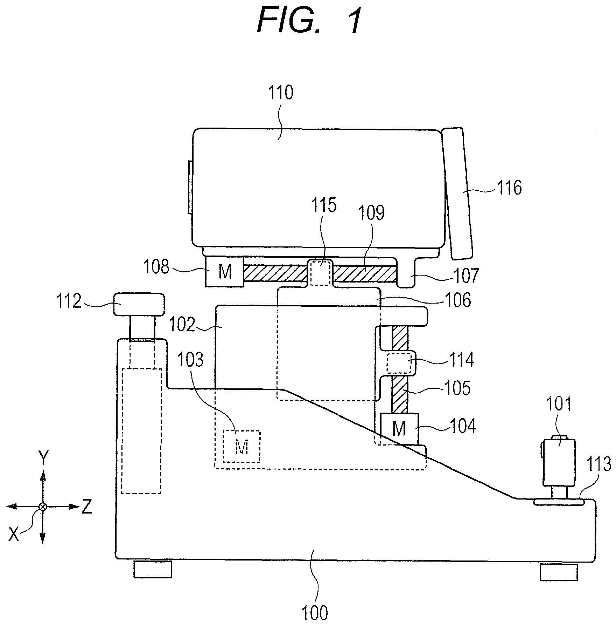

FIG. 1 is a schematic configuration diagram of an ophthalmologic reflectometer, which is an example of an ophthalmologic apparatus according to the present invention.

A frame 102 is movable in a left-right direction (X-axis direction of FIG. 1) with respect to a base 100. An X axis motor 103 is rotated to move the frame 102 in the left-right direction via a feed screw (not shown) and a nut (not shown). A frame 106 is movable in an up-down direction (Y-axis direction of FIG. 1) with respect to the frame 102. A Y axis motor 104 is rotated to move the frame 106 in the up-down direction via a feed screw 105 and a nut 114. A frame 107 is movable in a front-back direction (Z-axis direction of FIG. 1) with respect to the frame 106. A motor 108 is rotated to move the frame 107 in the front-back direction via a feed screw 109 and a nut 115.

On the frame 107, a measurement unit 110 for measurement is fixed. On the base 100, a joystick 101 for controlling a position of the measurement unit 110 is provided. Below the joystick 101, a jog dial 113 for setting a vertex distance by being rotated is provided.

When an eye refractive power is to be measured, a subject places his/her chin on a chin rest 112 and pushes his/her forehead to a forehead rest portion of a face rest frame (not shown) fixed to the base 100, and hence a position of an eye to be inspected can be fixed.

On an end portion of the measurement unit 110 on an inspector side, there is provided an LCD monitor 116 as a display member for observing an eye to be inspected E, which can display a measurement result and the like.

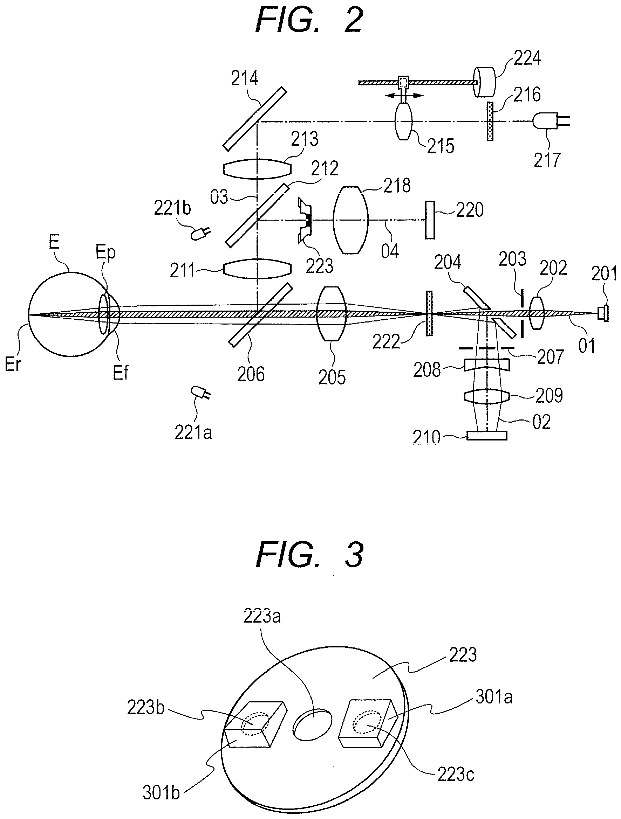

FIG. 2 is an arrangement diagram of an optical system arranged inside the measurement unit 110.

An optical path 01 from an eye refractive power measurement light source 201 emitting light having a wavelength of 880 nm to the eye to be inspected E is an optical axis of the reflectometer. On the optical path 01, there are arranged a lens 202, a stop 203 substantially conjugate with a pupil Ep of the eye to be inspected E, a perforated mirror 204, a diffuser panel 222 that can be inserted and extracted, a lens 205, and a dichroic mirror 206 that totally reflects visible light from the eye to be inspected E side and partially reflects a light beam having a wavelength of 880 nm, in this order. Note that, the wave length is not limited to the above-mentioned value.

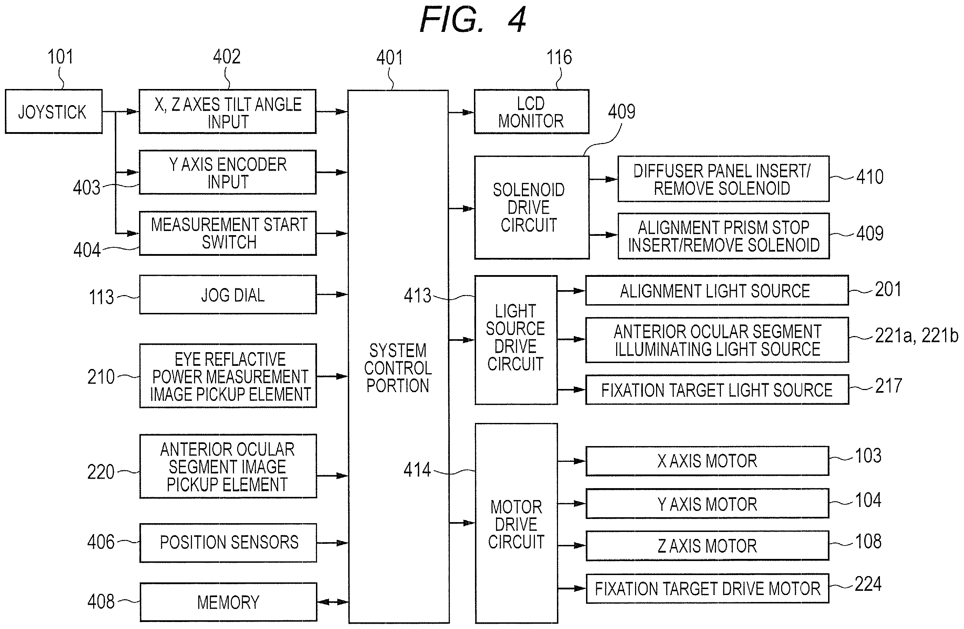

On an optical path 02 in the reflection direction of the perforated mirror 204, there are arranged an eye refractive power measurement stop 207, a light beam separation prism 208, a lens 209, and an image pickup element 210, in this order. When eye refractive power is to be measured, the translucent diffuser panel 222 is arranged outside the optical path by a diffuser panel insertion/extraction solenoid 410 (see FIG. 4). A light beam emitted from the measurement light source 201 is restricted by the stop 203 and primarily forms an image on the lens 202 before the lens 205. Then, after being transmitted through the lens 205 and the dichroic mirror 206, the light beam is projected to the pupil center of the eye to be inspected E.

The light beam forms an image on a fundus Er, and reflection light thereof is transmitted through the pupil center and enters the lens 205 again. The entering beam is transmitted through the lens 205 and then is reflected by a periphery of the perforated mirror 204.

The reflected beam is separated by pupil separation in the eye refractive power measurement stop 207 substantially conjugate with the pupil Ep of the eye to be inspected E and the beam separation prism 208. Because the eye refractive power measurement stop 207 has a ring-like slit, the light beam separated by pupil separation is projected as a ring image to a light receiving plane of the image pickup element 210.

When the eye to be inspected E is an emmetropic eye, this projected ring image becomes a predetermined circle. When the eye to be inspected E is a short-sighted eye, the projected circle becomes smaller than that in the emmetropic eye. When the eye to be inspected E is a long-sighted eye, the projected circle becomes larger than that in the emmetropic eye. When the eye to be inspected E has astigmatism, the projected ring image becomes an ellipse in which an angle formed between a horizontal axis and a major axis or a minor axis of the ellipse is an astigmatic axis angle. Based on a coefficient of this ellipse, the refractive power is determined.

On the other hand, in the reflection direction of the dichroic mirror 206, there are arranged a fixation target projecting optical system and an alignment light receiving optical system used for both anterior ocular segment observation and alignment detection of the eye to be inspected.

On an optical path 03 of the fixation target projecting optical system, there are arranged a lens 211, a dichroic mirror 212, a lens 213, a reflection mirror 214, a lens 215, a fixation target 216, and a fixation target light source 217 in the stated order.

When the fixation target control is performed, a projection light beam from the turned-on fixation target light source 217 illuminates the fixation target 216 from the backside, and is projected to the fundus Er of the eye to be inspected E via the lens 215, the reflection mirror 214, the lens 213, the dichroic mirror 212, and the lens 211. Note that, the lens 215 can be moved in an optical axis direction by a fixation target drive motor 224 which performs diopter drive control so as to realize a fogged state of the eye to be inspected E.

On an optical path 04 in the reflection direction of the dichroic mirror 212, there are arranged an alignment prism stop 223, a lens 218, and an image pickup element 220 in the stated order.

Light beams of an anterior ocular segment image of the eye to be inspected E illuminated by anterior ocular segment illuminating light sources 221a and 221b each having a wavelength of about 780 nm form images on the image pickup element 220 via the dichroic mirror 206, the lens 211, the dichroic mirror 212, and the alignment prism stop 223. The wavelength of the light emitted from each of the anterior ocular segment illuminating light sources 221a and 221b is not limited to the above-mentioned value.

When alignment is to be performed, the diffuser panel 222 is inserted by a diffuser panel insert/remove solenoid 410 (not shown) in the optical path. The insertion position is substantially the position at which the measurement light source 201 primarily forms the image by the projection lens 202, and is a focal position of the lens 205.

A light source for detecting the alignment is used also as the measurement light source 201 for measuring the eye refractive power described above. An image of the measurement light source 201 is once formed on the diffuser panel 222, and the image becomes a secondary light source so that the lens 205 projects a thick collimated light beam toward the eye to be inspected E. The collimated light beam is reflected by a cornea Ef of the eye to be inspected, and a part of the reflection light beam is reflected again by the dichroic mirror 206 and forms an image on the image pickup element 220 via the lens 211, the dichroic mirror 212, the alignment prism stop 223, and the lens 218.

FIG. 3 illustrates a shape of the alignment prism stop 223.

Three apertures 223a, 223b, and 223c are formed in a disk-shaped stop plate, and alignment prisms 301b and 301c, each of which transmits only a light beam having a wavelength of around 880 nm, are affixed to the apertures 223b and 223c on the dichroic mirror 212 side. The wavelength to be transmitted by each of the alignment prisms 301b and 301c is not limited to the above-mentioned value.

FIG. 4 is a system block diagram.

A basic flow of the eye refractive power measurement is described with reference to FIG. 4. A system control portion 401 that controls the entire system includes a program storage portion, a data storage portion, an input and output control portion for controlling input and output with various devices, and a calculation processing portion for calculating data obtained from various devices.

First, the system control portion 401 turns on the measurement light source 201, the anterior ocular segment illuminating light sources 221a and 221b, and the fixation target light source 217 via a light source drive circuit 413 to perform alignment and prepare for IOL eye determination and refractive power measurement.

The inspector operates the joystick 101 to align the measurement unit 110 with respect to the eye to be inspected E. On the joystick 101, a tilt angle detecting system 402 for forward, backward, left, and right tilts, an encoder input system 403 for rotations, a measurement start switch 404 that is pressed to start the measurement, and the jog dial 113 for changing the vertex distance are arranged. The system control portion 401 controls a motor drive circuit 414 to drive the X axis motor 103, the Y axis motor 104, and the Z axis motor 108 in response to inputs from the tilt angle detecting system 402 and the encoder input system 403 and hence controls the position of the measurement unit 110.

At the same time, the system control portion 401 combines the anterior ocular segment image of the eye to be inspected E, which is picked up by the anterior ocular segment image pickup element 220, with text and graphic data and displays the resulting image on the LCD monitor 116. The inspector rotates the jog dial 113 to select the vertex distance from 0.0 mm, 12.0 mm, and 13.5 mm. The selected vertex distance is displayed on the LCD monitor 116. Subsequently, the inspector performs the alignment so as to satisfy an alignment completion condition, which is to be described later, while watching the LCD monitor 116. Note that, the vertex distance is not limited to the above-mentioned values. Alternatively, the vertex distance may be selected from four or more values, or from two values. Further, instead of providing a plurality of vertex distances, a single vertex distance may be provided.

While the inspector is performing the alignment, the system control portion 401 automatically performs the IOL eye determination, which is to be described later. As a result, when the eye to be inspected is determined to be an IOL eye, the system control portion 401 displays the information on the LCD monitor 116 and switches the function of the jog dial 113 (see Japanese Patent No. 3244873). After the switching, the inspector uses the jog dial 113 to set the fixation target light source 217 to an appropriate amount of light.

When the alignment is complete, the inspector presses the measurement start switch 404 to transition to eye refractive power measurement.

In the eye refractive power measurement, the system control portion 401 retracts the diffuser panel 222, which has been inserted to the optical path 01, from the optical path 01 and projects a measurement light beam to the fundus Er of the eye to be inspected E.

The reflection light from the fundus propagates along the optical path 02 and is received by the eye refractive power measurement image pickup element 210. The fundus image is picked up in a ring shape by the eye refractive power measurement stop 207. This ring image is stored in a memory 408. Next, the system control portion 401 calculates barycentric coordinates of the ring image stored in the memory 408 to determine an ellipse equation by a well-known method. A long diameter, a short diameter, and a tilt angle of the major axis of the determined ellipse are calculated so that the eye refractive power of the eye to be inspected E is calculated to be displayed on the LCD monitor 116. Note that, an eye refractive power value corresponding to the determined long and short diameters of the ellipse, and a relationship between an angle of an ellipse axis on the light receiving surface of the image pickup element 210 and an astigmatic axis is calibrated in advance in a manufacturing process of the apparatus.

After the eye refractive power is obtained, the motor drive circuit 414 drives the fixation target drive motor 224 to move the lens 215 to a position corresponding to the refractive power value of the eye to be inspected E. Thereafter, the lens 215 is moved away by a predetermined amount so that the fixation target 216 fogs the eye to be inspected, and the measurement light source 201 is turned on again for the measurement of the eye refractive power. In this manner, the measurement of the eye refractive power and the fogging by the fixation target 216 are repeated, and at a stage where a measured value satisfies a predetermined termination condition, a true value of the eye refractive power may be obtained.

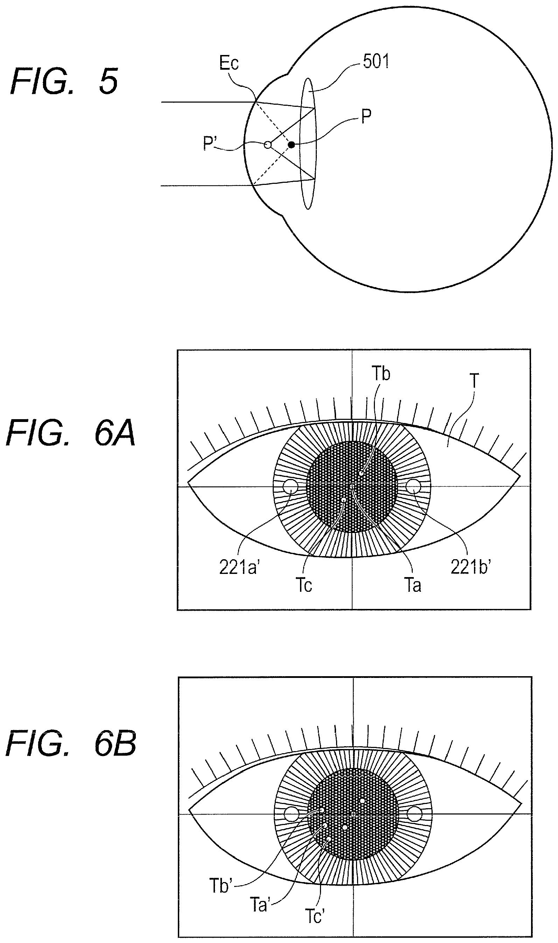

FIG. 5 illustrates cornea bright spots on an anterior ocular segment of the eye to be inspected. A part of the light beam from the measurement light source 201 is reflected by a cornea Ec so that a virtual image P is formed by corneal reflection. Another part of the projection light beam that is not reflected by the cornea is reflected by a crystalline lens or IOL 501 to form a real image P'. The image P' is formed at a position closer to the cornea than the image P. A crystalline lens in general has a refractive index close to that of a vitreous body and hence has a low reflectance on a back surface thereof, and hence the image P' is very dark as compared to the image P. On the other hand, the IOL has a significantly different refractive index than that of the vitreous body, and hence has a high reflectance on a back surface thereof. As a result, the image P' is lighter than in the case of the crystalline lens. Therefore, by identifying the lightness of the image P' formed by the measurement light source 201, it is possible to determine whether the eye to be inspected is the IOL eye. The measurement light source 201 may thus be used as an IOL eye determination light source.

FIGS. 6A and 6B illustrate examples of images picked up by the anterior ocular segment image pickup element 220.

FIG. 6A illustrates an image of a non-IOL eye, and FIG. 6B illustrates an image of the IOL eye. An anterior ocular segment image T in FIG. 6A is an image picked up of an anterior ocular segment of the eye to be inspected E illuminated by the anterior ocular segment illuminating light sources 221a and 221b. Bright spot images 221a' and 221b' are images picked up of reflections of the anterior ocular segment illuminating light sources 221a and 221b by the cornea Ef, respectively. The light beam from each of the anterior ocular segment illuminating light sources 221a and 221b has a wavelength of about 780 nm, and hence is transmitted through only the aperture 223a of the alignment prism stop. On the other hand, the light beam from the measurement light source 201 has a wavelength of about 880 nm, and hence is transmitted through all of the apertures 223a, 223b, and 223c of the alignment prism stop and refracted through the prisms 301b and 301c. Cornea bright spot images Ta, Tb, and Tc are the image P of FIG. 5 picked up after being transmitted through the apertures 223a, 223b, and 223c of the alignment prism stop, respectively.

The alignment prisms 301b and 301c have such refractive powers that the distance in the optical axis direction between the eye to be inspected and the apparatus when the cornea bright spot images Ta, Tb, and Tc are vertically aligned becomes the appropriate distance illustrated in FIG. 2. In addition, when the cornea bright spot image Ta is at the center of the anterior ocular segment image, the position of the eye to be inspected with respect to a direction perpendicular to the optical axis of the apparatus is an appropriate position illustrated in FIG. 2. Therefore, based on the positions of the cornea bright spot images Ta, Tb, and Tc, an alignment status between the apparatus and the eye to be inspected may be identified. This identification of the alignment status based on the corneal reflection image is executed by a module region functioning as an alignment status determining unit in the system control portion 401. The image pickup element 220 is set to such sensitivity that the image P of the non-IOL eye is picked up and the image P' thereof is not picked up.

FIG. 6B illustrates an image picked up of the IOL eye. In addition to the images of FIG. 6A, bright spot images Ta', Tb', and Tc' are picked up. Those images are the image P' of FIG. 5 picked up after being transmitted through the apertures 223a, 223b, and 223c of the alignment prism stop, respectively. The image pickup element is set to the same sensitivity as in FIG. 6A, but because the IOL has a high reflectance on the back surface thereof, the image P' is also picked up.

Depending on positions at which the image P and the image P' are formed, the bright spot images Ta and Ta' overlap each other and cannot be separated on the image pickup element in some cases. However, as illustrated in FIG. 5, the image P and the image P' are formed at different positions in the optical axis direction, and hence when refracted by the prisms 301b and 301c, the bright spot images are projected to the image pickup element 220 at different angles. Therefore, the bright spot images Tb and Tb' and Tc and Tc' do not overlap each other and can be identified at separate positions on the image pickup element 220.

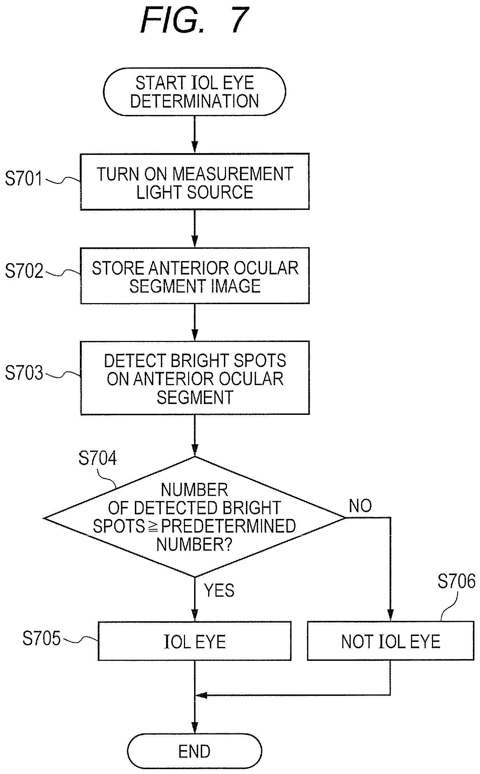

FIG. 7 illustrates a flow of the IOL eye determination. First in Step S701, the measurement light source 201 is turned on, and in Step S702, the anterior ocular segment image at the time is stored in the memory 408. The measurement light source 201 and an optical system for projecting the light beam from the light source to the eye to be inspected function here as a light beam projecting unit for projecting the light beam to the cornea of the eye to be inspected in the present invention. The image pickup element 220 for acquiring the anterior ocular segment image and an optical system used in association therewith to obtain the anterior ocular segment image function as a light receiving unit including the image pickup element for receiving the reflection light beam from the cornea in the present invention.

Next in Step S703, when included in a stored anterior ocular segment image A, the bright spot images Ta, Tb, Tc, Ta', Tb', and Tc' are detected. This operation is executed by a module region functioning as a bright spot image detecting unit in the system control portion 401. The detection is performed as follows.

The maximum brightness value of a certain region at the center of the anterior ocular segment image A is determined, and using a half value of the maximum brightness value as a threshold number, pixels having brightnesses higher than the threshold number are extracted. Then, areas of the extracted regions are determined, and regions having areas in a predetermined range are detected as the bright spot images.

When the eye to be inspected is not the IOL eye, the bright spot images Ta', Tb', and Tc' are not detected, and the number of bright spot images is at most 3. Therefore, in Step S704, the number of detected bright spot images is judged, and when the number is less than 4, it is determined in Step S706 that the eye to be inspected is not the IOL eye. When the number of detected bright spot images is 4 or more, it is determined in Step S705 that the eye to be inspected is the IOL eye. In other words, in this embodiment, based on the number of cornea bright spot images, it is determined whether or not the eye to be inspected is the IOL eye.

Through the above-mentioned flow, it is possible to determine whether the eye to be inspected is the IOL eye.

According to this embodiment, the automatic determination on whether the eye to be inspected is the IOL eye is enabled by utilizing the bright spot images on the cornea. Therefore, there is no risk that the inspection fails due to the mistake or the input error of the inspector, and because the input operation is unnecessary, the effects that the burden on the inspector is reduced and that the inspection time may be reduced may be obtained.

Further, it is possible to determine whether or not the eye to be inspected is the IOL eye by using an alignment light source, and hence there is no need to provide a special component for determining whether the eye to be inspected is the IOL eye.

Note that, the cornea bright spot images used in this embodiment are specular reflection images obtained by specular reflection on the cornea. The determination of a brightness of the image obtained by the specular reflection is easy, and hence by adding a component to be described below to the conventional configuration, the determination of the IOL eye may be performed with a simple configuration. Further, in the case where the above-mentioned Ta, Tb, and Tc are used as the cornea bright spot images and in other such cases, the threshold number used for the comparison with those bright spot images is stored by a module region functioning as a storage unit in the system control portion 401.

When the bright spot images are used, a module region functioning as an acquiring unit in the system control portion 401 acquires the threshold number of 4, for example, stored in the storage unit, and a module region functioning as a comparing unit compares the selected threshold number and the number of bright spot images actually detected by the bright spot image detecting unit. Those components constitute an IOL determining unit.

### [Second embodiment]

In the first embodiment, the measurement light source 201 functioning also as the alignment light source is used as the IOL eye determination light source. However, the IOL eye determination light source may be provided independently of the measurement light source. Further, the projection light beam emitted by the IOL eye determination light source may enter the eye to be inspected at a different angle from the optical axis of the apparatus. An ophthalmologic reflectometer having such configuration is described in a second embodiment of the present invention.

An arrangement diagram of an optical system in the second embodiment is similar to that of the first embodiment. However, the anterior ocular segment illuminating light source 221a or 221b, or both thereof are used instead of the measurement light source 201 as the IOL eye determination light source in the second embodiment. In this embodiment, those components constitute a light beam projecting unit. When an angle formed by the anterior ocular segment illuminating light sources 221a and 221b and the optical axis of the apparatus is large, a light beam emitted by the anterior ocular segment illuminating light source 221a or 221b may be blocked by an iris and may not reach the back surface of the IOL. Therefore, the accuracy of the IOL eye determination may be improved when the anterior ocular segment illuminating light sources 221a and 221b are arranged so that the angle formed with the optical axis of the apparatus becomes smaller than in the first embodiment. In other words, in the first embodiment, the optical axis of the light beam projected by the light beam projecting unit coincides with the optical axis of the reflection light beam, but in this embodiment, the light beam enters the eye to be inspected with the optical axis being at a different angle than the optical axis of the reflection light beam. Note that, in the present invention, the concept of "coincidence" includes not only a case where the optical axes completely coincide but also a case where the optical axes substantially coincide.

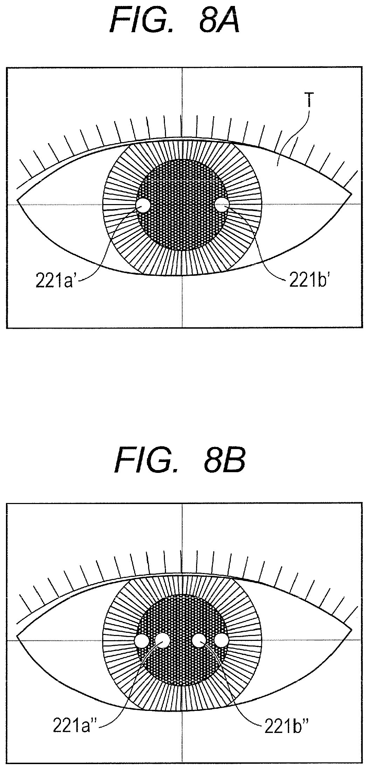

Examples of images picked up by the image pickup element 220 in the second embodiment are illustrated in FIGS. 8A and 8B.

FIG. 8A is a diagram illustrating a picked-up image of the non-IOL eye, and FIG. 8B is a diagram illustrating a picked-up image of the IOL eye. As in FIG. 6A, the bright spot images 221a' and 221b' are images of the light beams emitted by the anterior ocular segment illuminating light sources 221a and 221b and reflected on the cornea. When neither the alignment nor the eye refractive power measurement is performed, the measurement light source 201 may be turned off. FIGS. 8A and 8B illustrate cases where the measurement light source 201 is turned off. Bright spot images 221a'' and 221b'' are images of the light beams emitted by the anterior ocular segment illuminating light sources 221a and 221b and reflected on the back surface of the IOL. The bright spot images 221a'' and 221b'' are lighter than the reflection image on the back surface of the crystalline lens, and hence are picked up only in the case of the IOL eye. Further, the light beams emitted by the anterior ocular segment illuminating light sources 221a and 221b enter the eye to be inspected at different angles, and hence there is no fear that the bright spot images 221a'' and 221b'' are picked up as being entirely overlapped with the bright spot images 221a' and 221b'. Therefore, those bright spot images may be detected to determine whether the eye to be inspected is the IOL eye. Note that, the bright spot images 221a' and 221b' obtained by the reflection on the cornea may be used for alignment as with the bright spot images Ta, Tb, and Tc in the first embodiment.

The flow of the IOL eye determination may be similar to that of the first embodiment. It should be noted, however, that in the flow of the first embodiment, even for the eye to be inspected that is not the IOL eye, the image of ambient light reflected on the cornea may be incorrectly detected, and the eye to be inspected may be erroneously determined to be the IOL eye.

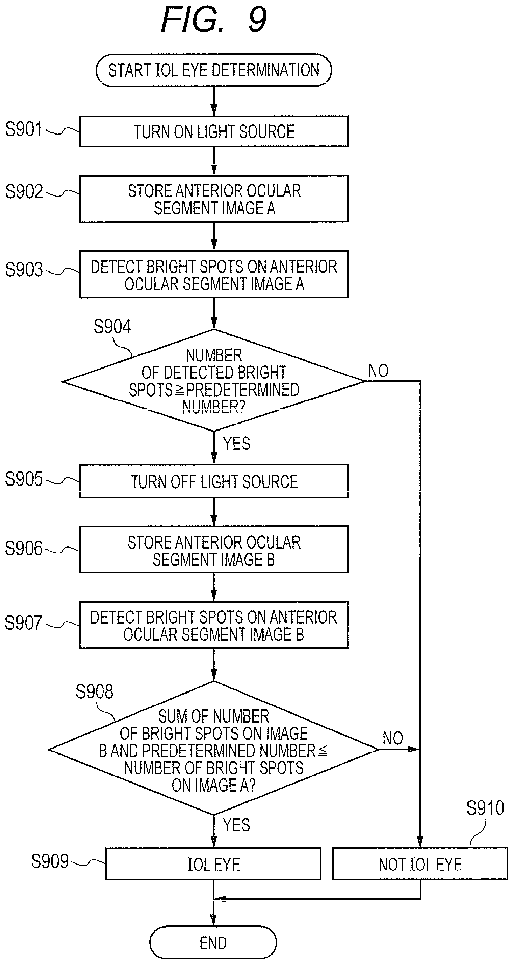

Therefore, in order to further improve the accuracy of the IOL eye determination, the determination may be performed through a flow as in FIG. 9. In FIG. 9, first in Step S901, the anterior ocular segment illuminating light sources 221a and 221b are turned on, and in Step S902, the anterior ocular segment image A at the time is stored in the memory 408. Next in Step S903, when included in the stored anterior ocular segment image A, bright spot images are detected. When the eye to be inspected is not the IOL eye, the bright spot images obtained by the reflection on the back surface of the IOL are not detected, and the number of bright spot images is at most 2.

Therefore, in Step S904, the number of detected bright spot images is judged, and when the number is less than 3, it is determined in Step S910 that the eye to be inspected is not the IOL eye. When the number of detected bright spot images is 3 or more, the processing proceeds to Step S905, in which the anterior ocular segment illuminating light sources 221a and 221b are turned off. In Step S906, an anterior ocular segment image B at the time is stored at a different address in the memory 408 than the anterior ocular segment image A. In Step S907, bright spot images on the anterior ocular segment image B are detected as in Step S903. In a case where the bright spot images detected on the anterior ocular segment image A are the bright spot images formed by the anterior ocular segment illuminating light sources 221a and 221b, when the anterior ocular segment illuminating light sources 221a and 221b are turned off, the bright spot images disappear and are not detected on the anterior ocular segment image B. However, in a case where the bright spot images are formed by the ambient light or the like, even when the anterior ocular segment illuminating light sources 221a and 221b are turned off, the bright spot images do not disappear and hence are detected also on the anterior ocular segment image B. Note that, instead of completely turning off the anterior ocular segment illuminating light sources 221a and 221b, the anterior ocular segment illuminating light sources 221a and 221b may be darkened to such an extent that the bright spot images cannot be picked up by the image pickup element 220.

Therefore, in Step S908, the numbers of bright spot images detected on the anterior ocular segment image A and the anterior ocular segment image B are compared, and when the number of bright spot images detected on the anterior ocular segment image B is smaller than the number of bright spot images detected on the anterior ocular segment image A by 3 or more, it is determined in Step S909 that the eye to be inspected is the IOL eye. Otherwise, it is determined in Step S910 that the eye to be inspected is not the IOL eye. In this manner, it is determined whether the eye to be inspected is the IOL eye. In other words, in this embodiment, an IOL determining unit compares the corneal reflection image obtained in the state in which the light beams are projected and the corneal reflection image obtained in the state in which the light beams are not projected to determine whether or not the eye to be inspected is the IOL eye. Further, the acquiring unit acquires the threshold number of 3, for example, stored in the storage unit. In other words, the acquiring unit changes the threshold number to be acquired depending on the bright spots used for the determination on whether or not the eye to be inspected is the IOL eye.

As described above, in this embodiment, not only similar effects as those of the first embodiment may be obtained, but also because the bright spot images in the state in which the anterior ocular segment illuminating light sources 221a and 221b are turned off are used, it is possible to determine whether the bright spot images are formed by the ambient light or the like or formed by the IOL. Therefore, the determination on whether or not the eye to be inspected is the IOL eye may be performed more accurately.

In the second embodiment, the anterior ocular segment illuminating light sources 221a and 221b are used as the light sources for the IOL eye determination, but a light source that is independent of all the measurement light sources, the light source for the alignment detection, and the anterior ocular segment illuminating light sources may be used instead.

The IOL eye determination may be performed with a condition other than the number of bright spot images. The image formed by the reflection on the back surface of the IOL is weaker than the image formed by the corneal reflection, and hence in the inspection of the IOL eye, an image of a different brightness than a normal eye is picked up on the image pickup element 220. Therefore, whether the eye to be inspected is the IOL eye may be determined based on the brightness of the image. Further, the image formed by the corneal reflection and the image formed by the reflection on the back surface of the IOL are formed at different positions, and hence in the inspection of the IOL eye, an image of a different size than the normal eye is picked up on the image pickup element 220. Therefore, whether the eye to be inspected is the IOL eye may be determined based on the size of the image.

Further, those flows may be used with a different optical system arrangement. In such case, the condition on the detection of the images, such as the number of detected bright spot images, needs to be changed to suit the optical system arrangement.

Further, the present invention is not limited to the ophthalmologic reflectometer. The IOL eye determination of the present invention may also be applied to an ophthalmologic apparatus other than the ophthalmologic reflectometer, such as an ophthalmologic image acquiring apparatus, an apparatus for measuring eye axial length, and OCT. The ophthalmologic image acquiring apparatus and the apparatus for measuring eye axial length may perform the determination of the IOL eye by the technologies described in Japanese Patent Application Laid-Open Nos. 2003-290146 and 2011-136109, but when implemented in combination with the present invention, the IOL eye determination may be performed with even higher accuracy.

Note that, the present invention is not limited to the above-mentioned embodiments, and various modifications and alterations may be made without departing from the spirit of the present invention. For example, the processing of FIG. 7 may be applied to the second embodiment. To be specific, when there is a bright spot other than the bright spot images 221a' and 221b', and when the number of bright spot images is 3 or more, it is determined that the eye to be inspected is the IOL eye.

Alternatively, the processing of FIG. 9 may be applied to the first embodiment. To be specific, when there is a bright spot other than the bright spot images Ta, Tb, and Tc, and when the number of bright spot images is 4 or more, the eye refractive power measurement light source 201 is turned off, and the processing of Steps S906 to S908 is performed. Note that, in Step S908, the numbers of bright spot images detected on the anterior ocular segment image A and the anterior ocular segment image B are compared, and when the number of bright spot images detected on the anterior ocular segment image B is smaller than the number of bright spot images detected on the anterior ocular segment image A by 4 or more, for example, it is determined in Step S909 that the eye to be inspected is the IOL eye.

### (Other modified examples)

Further, the present invention may also be realized by executing the following process. Specifically, software (program) for realizing the functions of the embodiments described above is supplied to a system or an apparatus via a network or an arbitrary type of storage medium, and a computer (CPU or MPU) of the system or the apparatus reads and executes the program.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

In order to automatically determine whether an eye to be inspected is an IOL eye by using bright spot images on a cornea for inspection at high accuracy, an ophthalmologic apparatus is provided with: a light beam projecting unit for projecting a light beam on the cornea of the eye to be inspected; a light receiving unit including an image pickup element for receiving a reflection light beam obtained by reflection of the light beam projected by the projecting unit to obtain cornea bright spot images from the cornea of the eye to be inspected; and an IOL eye determining unit for determining whether the eye to be inspected is the IOL eye based on the cornea bright spot images received by the light receiving unit.

## Claims

1. An ophthalmologic apparatus, comprising:
a projecting unit configured to project a light beam to an eye to be inspected; and
a determining unit configured to determine whether or not an image of a number of bright spots based on a reflection light beam obtained by reflection of the light beam from the eye to be inspected is a bright spot image generated by an intraocular lens.

2. An ophthalmologic apparatus according to claim 1, wherein the determining unit is configured to determine whether the image is the bright spot image generated by the intraocular lens or a bright spot image generated by a cornea of the eye to be inspected.

3. An ophthalmologic apparatus according to claim 2, wherein the determining unit is configured to determine whether the image is the bright spot image generated by the intraocular lens or the bright spot image generated by the cornea of the eye to be inspected based on brightness of the image.

4. An ophthalmologic apparatus according to claim 2, wherein the determining unit is configured to determine whether the image is the bright spot image generated by the intraocular lens or the bright spot image generated by the cornea of the eye to be inspected based on a position at which the image is formed.

5. An ophthalmologic apparatus according to claim 1,
wherein the determining unit comprises:
an intraocular lens eye determining unit configured to determine whether or not the eye to be inspected is an intraocular lens eye; and
a bright spot image detecting unit configured to detect the image of the number of the bright spots, and
wherein the intraocular lens eye determining unit is configured to determine whether the eye to be inspected is the intraocular lens eye based on the number of the bright spots.

6. An ophthalmologic apparatus according to claim 5, wherein the intraocular lens eye determining unit is configured to compare the number of the bright spots detected by the bright spot image detecting unit and a threshold number.

7. An ophthalmologic apparatus according to claim 6, wherein the intraocular lens eye determining unit is configured to determine that the eye to be inspected is the intraocular lens eye when the number of the bright spots is larger than the threshold number.

8. An ophthalmologic apparatus according to claim 1, further comprising an alignment status determining unit configured to determine an alignment status between the ophthalmologic apparatus and the eye to be inspected based on the image.

9. An ophthalmologic apparatus according to claim 5, wherein the intraocular lens eye determining unit is configured to compare the bright spots obtained in a state in which the light beam is projected and the bright spots obtained in a state in which the light beam is not projected to determine whether the eye to be inspected is the intraocular lens eye.

10. An ophthalmologic apparatus according to claim 1, wherein an optical axis of the light beam projected by the projecting unit coincides with an optical axis of the reflection light beam.

11. An ophthalmologic apparatus according to claim 1, wherein the light beam projected by the projecting unit enters the eye to be inspected at a different angle than an optical axis of the reflection light beam.

12. An ophthalmologic apparatus according to claim 1, further comprising an acquiring unit configured to acquire a refractive power of the eye to be inspected based on the reflection light beam obtained by the reflection of the light beam, which is projected to the eye to be inspected by the projecting unit, from the eye to be inspected.

13. An ophthalmologic method for acquiring an image of an eye to be inspected, comprising:
projecting a light beam to the eye to be inspected; and
determining whether or not an image of a number of bright spots based on a reflection light beam obtained by reflection of the light beam from the eye to be inspected is a bright spot image generated by an intraocular lens.

## Patentansprüche

1. Ophthalmologische Vorrichtung mit
einer Projektionseinheit zum Projizieren eines Laserstrahls zu einem zu untersuchenden Auge und
einer Bestimmungseinheit zur Bestimmung, ob ein Bild einer Anzahl heller Flecke beruhend auf einem Reflexionslichtstrahl, der durch Reflexion des Lichtstrahls von dem zu untersuchenden Auge erhalten wird, ein durch eine Intraokularlinse erzeugtes Heller-Fleck-Bild ist oder nicht.

2. Ophthalmologische Vorrichtung nach Anspruch 1, wobei die Bestimmungseinheit zur Bestimmung eingerichtet ist, ob das Bild das durch die Intraokularlinse erzeugte Heller-Fleck-Bild oder ein durch eine Kornea des zu untersuchenden Auges erzeugtes Heller-Fleck-Bild ist.

3. Ophthalmologische Vorrichtung nach Anspruch 2, wobei die Bestimmungseinheit zur Bestimmung, ob das Bild das durch die Intraokularlinse erzeugte Heller-Fleck-Bild oder das durch die Kornea des zu untersuchenden Auges erzeugte Heller-Fleck-Bild ist, beruhend auf einer Helligkeit des Bildes eingerichtet ist.

4. Ophthalmologische Vorrichtung nach Anspruch 2, wobei die Bestimmungseinheit zur Bestimmung, ob das Bild das durch die Intraokularlinse erzeugte Heller-Fleck-Bild oder das durch die Kornea des zu untersuchenden Auges erzeugte Heller-Fleck-Bild ist, beruhend auf einer Position eingerichtet ist, an der das Bild erzeugt wird.

5. Ophthalmologische Vorrichtung nach Anspruch 1,
wobei die Bestimmungseinheit umfasst
eine Intraokularlinsenauge-Bestimmungseinheit zur Bestimmung, ob das zu untersuchende Auge ein Intraokularlinsenauge ist oder nicht, und
eine Heller-Fleck-Bild-Erfassungseinheit zur Erfassung des Bildes der Anzahl der hellen Flecke, und
wobei die Intraokularlinsenauge-Bestimmungseinheit zur Bestimmung, ob das zu untersuchende Auge das Intraokularlinsenauge ist, beruhend auf der Anzahl der hellen Flecke eingerichtet ist.

6. Ophthalmologische Vorrichtung nach Anspruch 5, wobei die Intraokularlinsenauge-Bestimmungseinheit zum Vergleichen der durch die Heller-Fleck-Bild-Erfassungseinheit erfassten Anzahl der hellen Flecke und einer Schwellenwertanzahl eingerichtet ist.

7. Ophthalmologische Vorrichtung nach Anspruch 6, wobei die Intraokularlinsenauge-Bestimmungseinheit zur Bestimmung, dass das zu untersuchende Auge das Intraokularlinsenauge ist, eingerichtet ist, wenn die Anzahl der hellen Flecke größer als die Schwellenwertanzahl ist.

8. Ophthalmologische Vorrichtung nach Anspruch 1, ferner mit einer Ausrichtungsstatusbestimmungseinheit zur Bestimmung eines Ausrichtungsstatus zwischen der ophthalmologischen Vorrichtung und dem zu untersuchenden Auge beruhend auf dem Bild.

9. Ophthalmologische Vorrichtung nach Anspruch 5, wobei die Intraokularlinsenauge-Bestimmungseinheit zum Vergleichen der hellen Flecke, die in einem Zustand erhalten werden, in dem der Lichtstrahl projiziert wird, und der hellen Flecke, die in einem Zustand erhalten werden, in dem der Lichtstrahl nicht projiziert wird, zur Bestimmung eingerichtet ist, ob das zu untersuchende Auge das Intraokularlinsenauge ist.

10. Ophthalmologische Vorrichtung nach Anspruch 1, wobei eine optische Achse des durch die Projektionseinheit projizierten Lichtstrahls mit einer optischen Achse des Reflexionslichtstrahls übereinstimmt.

11. Ophthalmologische Vorrichtung nach Anspruch 1, wobei der durch die Projektionseinheit projizierte Lichtstrahl in das zu untersuchende Auge an einem von einer optischen Achse des Reflexionslichtstrahls verschiedenen Winkel eintritt.

12. Ophthalmologische Vorrichtung nach Anspruch 1, ferner mit einer Beschaffungseinheit zur Beschaffung einer Brechkraft des zu untersuchenden Auges beruhend auf dem Reflexionslichtstrahl, der durch die Reflexion des Lichtstrahls, der durch die Projektionseinrichtung zu dem zu untersuchenden Auge projiziert wird, von dem zu untersuchenden Auge erhalten wird.

13. Ophthalmologisches Verfahren zum Beschaffen eines Bildes eines zu untersuchenden Auges, mit
Projizieren eines Lichtstrahls zu dem zu untersuchenden Auge und
Bestimmen, ob ein Bild einer Anzahl heller Flecke beruhend auf einem Reflexionslichtstrahl, der durch Reflexion des Lichtstrahls von dem zu untersuchenden Auge erhalten wird, ein durch eine Intraokularlinse erzeugtes Heller-Fleck-Bild ist oder nicht.

## Revendications

1. Appareil ophtalmologique, comprenant :
une unité de projection configurée pour projeter un faisceau lumineux vers un oeil à examiner ; et
une unité de détermination configurée pour déterminer si une image d'un nombre de points lumineux basés sur un faisceau lumineux de réflexion obtenu par réflexion du faisceau lumineux à partir de l'oeil à examiner est, ou non, une image de points lumineux générée par une lentille intraoculaire.

2. Appareil ophtalmologique selon la revendication 1, dans lequel l'unité de détermination est configurée pour déterminer si l'image est, ou non, l'image de points lumineux générée par la lentille intraoculaire ou une image de points lumineux générée par la cornée de l'oeil à examiner.

3. Appareil ophtalmologique selon la revendication 2, dans lequel l'unité de détermination est configurée pour déterminer si l'image est, ou non, l'image de points lumineux générée par la lentille intraoculaire ou l'image de points lumineux générée par la cornée de l'oeil à examiner sur la base de la luminosité de l'image.

4. Appareil ophtalmologique selon la revendication 2, dans lequel l'unité de détermination est configurée pour déterminer si l'image est, ou non, l'image de points lumineux générée par la lentille intraoculaire ou l'image de points lumineux générée par la cornée de l'oeil à examiner sur la base d'une position à laquelle est formée l'image.

5. Appareil ophtalmologique selon la revendication 1, dans lequel l'unité de détermination comprend :
une unité de détermination d'oeil doté d'une lentille intraoculaire configurée pour déterminer si l'oeil à examiner est, ou non, un oeil doté d'une lentille intraoculaire ; et
une unité de détection d'image de points lumineux configurée pour détecter l'image du nombre de points lumineux, et
dans lequel l'unité de détermination d'oeil doté d'une lentille intraoculaire est configurée pour déterminer si l'oeil à examiner est, ou non, l'oeil doté d'une lentille intraoculaire sur la base du nombre des points lumineux.

6. Appareil ophtalmologique selon la revendication 5, dans lequel l'unité de détermination d'oeil doté d'une lentille intraoculaire est configurée pour comparer le nombre des points lumineux détectés par l'unité de détection d'image de points lumineux et un nombre seuil.

7. Appareil ophtalmologique selon la revendication 6, dans lequel l'unité de détermination d'oeil doté d'une lentille intraoculaire est configurée pour déterminer que l'oeil à examiner est l'oeil doté d'une lentille intraoculaire lorsque le nombre des points lumineux est supérieur au nombre seuil.

8. Appareil ophtalmologique selon la revendication 1, comprenant en outre une unité de détermination d'état d'alignement configurée pour déterminer un état d'alignement entre l'appareil ophtalmologique et l'oeil à examiner sur la base de l'image.

9. Appareil ophtalmologique selon la revendication 5, dans lequel l'unité de détermination d'oeil doté d'une lentille intraoculaire est configurée pour comparer les points lumineux obtenus dans un état dans lequel le faisceau lumineux est projeté et les points lumineux obtenus dans un état dans lequel le faisceau lumineux n'est pas projeté de façon à déterminer si l'oeil à inspecter est, ou non, l'oeil doté d'une lentille intraoculaire.

10. Appareil ophtalmologique selon la revendication 1, dans lequel un axe optique du faisceau lumineux projeté par l'unité de projection coïncide avec un axe optique du faisceau lumineux de réflexion.

11. Appareil ophtalmologique selon la revendication 1, dans lequel le faisceau lumineux projeté par l'unité de projection pénètre l'oeil à examiner à un angle différent d'un axe optique du faisceau lumineux de réflexion.

12. Appareil ophtalmologique selon la revendication 1, comprenant en outre une unité d'acquisition configurée pour acquérir une réfringence de l'oeil à examiner sur la base du faisceau lumineux de réflexion obtenu par la réflexion du faisceau lumineux, qui est projeté vers l'oeil à examiner par l'unité de projection, à partir de l'oeil à examiner.

13. Méthode ophtalmologique d'acquisition d'une image d'un oeil à examiner, consistant à :
projeter un faisceau lumineux vers l'oeil à examiner ; et
déterminer si une image d'un nombre de points lumineux basés sur un faisceau lumineux de réflexion obtenu par réflexion du faisceau lumineux à partir de l'oeil à examiner est, ou non, une image de points lumineux générée par une lentille intraoculaire.
